# FASCICULE DE BREVET EUROPEEN

(11) **EP 0 863 763 B1**
(45) Date de publication et mention de la délivrance du brevet: **30.03.2005**
(21) Numéro de dépôt: 96939981.5
(22) Date de dépôt: 26.11.1996
(51) Int. Cl.: A61K 35/74, A23K 1/00, A23L 1/03

(54) **UTILISATION D'UNE PREPARATION DESHYDRATEE DE BACTERIES PROPIONIQUES POUR LA PREPARATION D'UNE COMPOSITION DIETETIQUE SUSCEPTIBLE D'AMELIORER L'EQUILIBRE BIOLOGIQUE DE LA FLORE DU TRACTUS INTESTINAL**
VERWENDUNG VON EINER DEHYDRIERTEN ZUBEREITUNG VON PROPIONISCHEN BAKTERIEN ZUR HERSTELLUNG EINER DIÄTETISCHEN ZUSAMMENSETZUNG ZUR VERBESSERUNG DES BIOLOGISCHEN GLEICHGEWICHTS DER DARMFLORA
USE OF A DEHYDRATED PREPARATION CONTAINING PROPIONIC BACTERIA FOR THE MANUFACTURE OF A DIETARY COMPOSITION FOR IMPROVING THE BIOLOGICAL BALANCE OF INTESTINAL TRACT FLORA

(30) Priorité: 27.11.1995 FR 9514019
(43) Date de publication de la demande: 16.09.1998
(73) Titulaire: Laboratoires Standa S.A., 14050 Caen Cédex (FR)
(72) Inventeur: ROUSSEL, Edmond, F-14210 Avenay (FR); LEGRAND, Charles, F-14000 Caen (FR)
(74) Mandataire: Cabinet HERRBURGER
(86) Numéro de dépôt international: PCT/FR1996/001871
(87) Numéro de publication internationale: WO 1997/019689

(56) Documents cités:
- WO-A-94/03195
- US-A- 4 806 368
- US-A- 5 501 857
- DATABASE WPI Week 9313 Derwent Publications Ltd., London, GB; AN 93-103614 XP002025755 & JP 05 041 995 A (MEIJI MILK PROD. CO. LTD.) , 23 Février 1993
- DATABASE WPI Week 7509 Derwent Publications Ltd., London, GB; AN 75-15346W XP002025756 & JP 50 003 397 B (MEIJI MILK PRODUCTS CO.) , 4 Février 1975

## Description

La présente invention concerne l'utilisation d'une préparation déshydratée de bactéries propioniques pour l'obtention d'une composition diététique absorbable susceptible d'améliorer l'équilibre biologique de la flore du tractus intestinal de l'homme et des mammifères.

Il est bien connu que le contenu du tube digestif humain, qui correspond approximativement à 1 à 1,5 kg de matières alimentaires en cours de transformation digestive, renferme une importante population de micro-organismes pouvant être évaluée à environ 10¹⁰ cellules/g dans le côlon.

Cette population est répartie en différents groupes bactériens dont certains sont inoffensifs ou bénéfiques, tandis que d'autres, en particulier des coliformes et des putréfiants conduisent à la production de substances toxiques et influencent négativement la santé.

Or, et notamment depuis le début du vingtième siècle, différents travaux ont tenté de montrer que la présence d'une population importante de bactéries lactiques limite très fortement le développement des espèces putréfiantes et par suite la production de ces matières toxiques.

Il a donc tout naturellement été proposé d'introduire dans l'organisme une population importante de ces bactéries, soit par le biais d'une alimentation particulière, soit par l'ingestion directe de ces cellules microbiennes ; parmi la flore lactique possible, on a notamment conseillé l'absorption des bactéries suivantes mentionnées à titre d'exemple non limitatif *Lactobacillus bulgaricus, Lactobacillus* acidophilus ou encore *Streptococus faecium.*

Cette administration n'a, toutefois, pas permis d'aboutir aux résultats escomptés compte tenu, d'une part, du fait que la population microbienne du tractus intestinal constitue une masse dont l'équilibre ne peut que difficilement être modifié radicalement et durablement et, d'autre part, que l'on a pu établir que la consommation de bactéries lactiques n'a que peu d'influence sur la flore putride qui provoque les malaises et désagréments connus.

Plus récemment, c'est-à-dire durant la décennie 1970/1980, on a également proposé d'introduire dans l'alimentation humaine des espèces non lactiques du type bifide qui, comme on l'a démontré, sont susceptibles de présenter un intérêt pour la santé. De tels ferments peuvent être consommés soit par le biais de desserts lactés ou autres produits laitiers fermentés pour partie par une flore bifide soit à travers l'ingestion directe de cellules vivantes conditionnées à cet effet.

Une telle introduction s'est cependant révélée décevante à l'usage, compte tenu en particulier du fait que les bactéries bifides exogènes ne réussissent que difficilement à s'implanter durablement dans l'intestin humain, notamment chez des sujets pour lesquels l'équilibre intestinal n'est pas des meilleurs.

Partant de ces connaissances d'ordre général, l'idée à la base de l'invention a consisté à chercher un moyen permettant d'augmenter la population bifide.

Ce faisant, on a été amené à prendre en considération les travaux du Professeur Henri Berrens de Lille qui a pu établir que l'acide propionique est un élément sélectif et électif spécifique de la flore bifide.

Or, il est connu que cet acide organique est l'un des produits résultant de la fermentation notamment du lactate par un type particulier de bactéries, les bactéries propioniques qui, bien que n'appartenant pas au groupe des bactéries lactiques, sont néanmoins présentes en alimentation humaine depuis des siècles, vu en particulier que ce sont elles qui permettent l'obtention des trous lors de la fabrication du fromage dénommé « Emmental » : en effet, en fin d'affinage, ces fromages renferment environ 10⁹ cellules/g de bactéries propioniques.

Il est à noter, qu'outre l'acide propionique, la fermentation des bactéries propioniques produit entre autre de l'acide acétique et du dioxyde de carbone.

Parallèlement à ces travaux, on a pu établir conformément à la publication TSUTOMU KANEKO, HIROHARU MORI, MEGUMI IWATA and SACHIKI MEGURO « Growth stimulator for Bifidobacteria Produced by *Propionibacterium freudenreichii* and Several Intestinal Bacteria » 1994 J; Dairy Sci 77:393-404, d'une part que des bactéries propioniques, et notamment des bactéries du type *Propionibacterium freundenreichii* peuvent produire un facteur de croissance des bactéries bifides et, d'autre part, que les acides gras à chaîne courte et en particulier l'acide propionique ont une action inhibitrice très importante sur la croissance de nombreuses bactéries intestinales indésirables mais stimulent la croissance des bactéries bifides.

Il a également déjà été proposé, conformément au document JP-A-07 227 207, d'ajouter des bactéries propioniques à des denrées alimentaires notamment à du lait fermenté renfermant des bactéries bifides dans le but d'augmenter la survie de ces bactéries.

Il est, en outre, à noter que l'on connaît, par le document US-A-4 806 368, un comprimé diététique à base de fibres alimentaires, en particulier de fibres de pomme pouvant renfermer des bactéries bifides et, en outre, une quantité moindre de bactéries propioniques agissant en tant qu'agent antifongique.

Partant de ces connaissances préalables, on a eu l'idée, conformément à l'invention, de tenter de faire consommer directement, et en grande quantité, des ferments bifides et propioniques associés dans le cadre d'une présentation dosée et prête à être ingérée facilement.

Or, on s'est rendu compte qu'une telle absorption s'avère en tout point bénéfique et permet en particulier de réduire de façon significative les manifestations néfastes habituelles des germes putrides, ce alors même que l'on aurait pu s'attendre à l'effet inverse, compte tenu de l'important dégagement de dioxyde de carbone consécutif à la fermentation propionique, comme l'atteste le rôle de ces ferments dans l'ouverture des fromages.

L'invention a, par suite, pour objet l'utilisation d'une préparation déshydratée renfermant des bactéries propioniques en quantité d'au moins 10⁹ cellules/g de préférence d'environ 10¹⁰ à 10¹² cellules/g pour l'obtention d'une composition diététique absorbable susceptible d'améliorer l'équilibre biologique de la flore du tractus intestinal de l'homme et des mammifères en stimulant le développement des bactéries bifides dans l'intestin.

La composition ainsi obtenue est donc très concentrée en bactéries.

Selon une autre caractéristique de l'invention la composition renferme également des bactéries bifides.

Ces bactéries sont, dans le cas général, des bactéries vivantes, mais peuvent également être tuées, à la condition que l'on ait pris soin de ne pas détruire leur capital enzymatique.

Ces bactéries sont, de préférence, réparties en 80 à 99 % de bactéries propioniques et 20 à 1 % de bactéries bifides.

La composition obtenue conformément à l'invention renferme donc avantageusement en moyenne dix fois plus de bactéries propioniques que de bactéries bifides.

Les bactéries bifides mises en oeuvre appartiennent, en règle générale, à l'espèce *Bifidobacterium* et sont, de préférence, choisies parmi les souches *B.bifidum, B.longum, B*.*adolescentis, B*.*breve, B*.*infantis* et *B.pseudolongum.*

Les bactéries propioniques appartiennent, quant à elles en règle générale, aux espèces *Propionibacterium freudenreichii* ou *Propionibacterium shermanii* ou *Propionibacterium thoenii ou Propionibacterium jensenii ou Propionibacterium acidipropionici.*

Ces bactéries peuvent être mises en oeuvre en souche pure, ou de préférence, en mélange de souches ; dans le cas des bactéries propioniques, il est avantageux d'associer simultanément des souches fortement autolytiques et des souches peu autolytiques.

Il est à noter que la population bifide dont le développement est stimulé peut provenir soit de bactéries endogènes soit de bactéries exogènes ajoutées à la préparation.

La composition obtenue conformément à l'invention se présente, de préférence, sous forme de fractions individuelles d'environ 100 mg à 1 g, de préférence 200 à 500 mg renfermant la dose de bactéries devant être absorbée quotidiennement.

Ces fractions peuvent être ingérées directement ou être préalablement diluées dans un liquide ; elles peuvent être conditionnées sous toute forme permettant de faciliter l'absorption : comprimés, sachets de poudre granulée ou non, ...

On a vérifié que de telles préparations déshydratées concentrées de bactéries propioniques conservées deux années à +4°C voient leur concentration baisser de moins de un Log.

L'expérience a prouvé que des gélules gastrorésistantes ou non correspondent à un type de conditionnement particulièrement avantageux.

Selon une autre caractéristique de l'invention, chaque fraction individuelle renferme au moins 10⁹ bactéries et de préférence de l'ordre de 10¹⁰ à 10¹² bactéries.

Il est à noter qu'en deçà du seuil de 10⁹ bactéries, l'effet est aléatoire, ce qui est, selon toute probabilité, consécutif au fait que les bactéries se trouvent alors détruites par l'acidité stomacale ou par les sels biliaires avant leur arrivée dans le côlon.

Selon l'invention, il est donc proposé de faire absorber quotidiennement par l'être humain, à titre de complément alimentaire, une dose de bactéries propioniques correspondant environ au millième de la flore totale du tube digestif (qui représente environ 10¹⁴ unités) ce qui correspond à une quantité nettement supérieure à celle proposée auparavant pour des bactéries de type autre.

On a pu vérifier qu'une telle administration conforme à l'invention est dénuée de tout effet néfaste et entraîne, outre les avantages susmentionnés :
- une diminution notable de l'émission de gaz malodorants typiques des putréfiants,
- une modification de la couleur et de l'odeur des selles.

Ces constatations sont clairement de nature à mettre en lumière l'activité « symbiotique » des bactéries propioniques et des bactéries bifides, les propioniques stimulant le développement des bifides.

Ce résultat a été confirmé par une étude réalisée dans le but de vérifier si des souches de Propionibacterium pouvaient soit améliorer la vitesse d'établissement d'une souche de *Bifidobacterium bifidum* dans le tube digestif de souris, soit augmenter son niveau d'activité dans les différents segments du tube digestif.

Cette étude a, dans un premier temps, montré qu'il n'est pas possible d'établir durablement des *Propionibacterium* dans le tube digestif de souris, même de souris sans germe, c'est-à-dire en l'absence de flore digestive.

On a, en revanche, pu montrer que l'apport de Pro*pionibacterium* en culture dans l'eau de boisson de ces souris permet de stimuler la vitesse d'implantation de souches de *Bifidobacterium bifidum* dans le tube digestif.

Cette étude scientifique apporte donc la preuve qu'il existe, une réelle synergie entre les actions des bactéries propioniques et des bactéries bifides.

Ces résultats ont, en outre, pu être vérifiés par une série d'investigations réalisées, d'une part, in vivo chez l'homme sain et, d'autre part, in vitro.

### Investigations in vivo

Celles-ci ont eu pour but l'étude en milieu hospitalier au CHU de Caen de la survie et/ou de l'implantation de souches de bactéries propioniques dans le milieu intestinal et leur effet sur la flore bifide d'une série de dix-neuf sujets masculins volontaires sains.

Conformément à cette étude, on a fait ingérer quotidiennement à chaque volontaire, ce pendant deux semaines, une gélule contenant 5 10¹⁰ bactéries propioniques sélectionnées par l'INRA et issues d'une banque de souches utilisées dans l'industrie fromagère, donc parfaitement inoffensives pour l'homme.

Les bactéries propioniques administrées se subdivisaient en deux souches à raison d'environ 50 % chacune, l'une de ces souches étant peu autolytique tandis que l'autre était sensée s'autolyser au niveau de l'estomac et au début de l'intestin grêle sous l'action des sels biliaires.

L'étude de la flore fécale a été réalisée sur un échantillon de selles avant la prise de propioniques pour avoir l'état basal de la flore, au cours de cette prise et postérieurement à celle-ci pour apprécier la durée de survie des bactéries exogènes dans le tube digestif des sujets volontaires.

Cette étude a inclus une numération des propioniques ingérés et des bifidobactéries.

Suite à ces investigations, il a pu être vérifié que la population de bactéries propioniques dans les selles augmentait significativement dès le premier prélèvement sous propioniques mais que cet effet est transitoire et cesse peu après l'arrêt de l'ingestion des bactéries.

Ces résultats montrent qu'une fraction des souches de bactéries propioniques peut survivre dans le tube digestif humain, mais que celles-ci ne peuvent pas s'y implanter durablement.

Pour apprécier l'effet des bactéries propioniques sur la population bifide, on a également pris en considération des prélèvements effectués avant l'ingestion des propioniques (a), pendant cette ingestion (b) et après celle-ci (deux prélèvements (c et d) effectués à une semaine d'intervalle).

Il est à noter que le prélèvement (b) correspond à la moyenne de deux prélèvements effectués à une semaine d'intervalle.

Une numération des Bifidobactéries a permis de calculer les moyennes (en Log) des populations pendant ces quatre périodes.

On a ainsi trouvé des valeurs moyennes respectives de 8,40 (a) ; 8,90 (b) ; 9,43 (c) et 8,89 (d). L'évolution avec le temps a été étudiée par un test statistique non paramétrique pour populations appariées, le Wilcoxon Matched-paired Signed-Ranks Test. Il montre que lors de l'ingestion de propioniques (prélèvement b) la population bifide augmente significativement par rapport au chiffre de base (prélèvement a) (p=0,0096) et reste élevée au premier contrôle (prélèvement c) après l'arrêt de leur ingestion (p=0,031). Il n'y a pas de différence entre la numération de Bifidobactéries pendant l'apport de propioniques (prélèvement b) et ce premier contrôle après son arrêt (1 semaine) (prélèvement c) (p=0,19) ; la population de Bifidobactéries diminue au deuxième contrôle (prélèvement d) (p=0,049) en comparaison avec le premier contrôle après arrêt (prélèvement c) et revient au niveau antérieur à l'apport des propioniques (prélèvement a) (p=0,25) entre le 2ème contrôle (prélèvement d) et le prélèvement le précédant (prélèvement c).

Ces résultats montrent que l'apport à dose élevée de propioniques favorise le développement de la flore bifide ; cet effet est rapide, observé dès la première semaine et persiste au moins une semaine après l'arrêt de l'ingestion des propioniques.

### Investigations in vitro

Le but de ces investigations était :
- d'étudier la résistance des bactéries propioniques au « stress » digestif, c'est-à-dire l'acidité stomacale et les sels biliaires,
- d'étudier l'influence de cultures de bactéries propioniques sur la croissance des bifidobactéries,
- de caractériser, le cas échéant, l'agent responsable de cette influence.

### Résistance des bactéries propioniques au « stress » digestif

Entre leur ingestion et leur arrivée dans le côlon où elles entrent en contact avec les bactéries intestinales et notamment les bactéries bifides, les bactéries propioniques subissent un certain nombre de « stress » défavorables à leur survie, dont les principaux sont l'acidité stomacale et le contact avec les sels biliaires dans l'intestin grêle.

Pour apprécier la résistance des bactéries propioniques à ces « stress », on a choisi deux souches appartenant à l'espèce *Propionibacterium freudenreichii,* à savoir la souche LS 410 qui est peu autolytique et la souche LS 2501 qui est fortement autolytique.

Chez l'homme le pH stomacal, après l'arrivée du bol alimentaire, se situe entre 2 et 3 et le temps moyen de vidange gastrique est de l'ordre de 90 min..

Pour évaluer la résistance des souches susmentionnées à l'acidité stomacale, on a donc testé celles-ci à différents pH (2, 3 et 4) pendant 90 min. à 37°C.

On a pu faire les observations suivantes :
A pH 4, la viabilité des deux souches n'est pas du tout altérée.
A pH 3, la souche LS 410 n'est pas endommagée mais la souche LS 2501 présente une légère baisse de viabilité.
A pH 2, la quasi totalité des deux souches meurt après 90 min. d'incubation.

La résistance des deux souches aux sels biliaires à 37°C a été évaluée en choisissant des concentrations d'acides biliaires de 1, 2 et 5 g/l correspondant essentiellement à la concentration à l'entrée de l'intestin grêle.

Ces investigations ont permis de constater qu'à la concentration la plus faible (1 g/l) la souche LS 410 reste entièrement résistante même après 5 h 30 d'incubation. En revanche, la souche LS 2501 présente une moindre résistance.

En présence de 2 et 5 g/l d'acides biliaires, la viabilité des deux souches chute nettement, la souche LS 2501 étant plus sensible que la souche LS 410.

En conclusion, ces investigations ont montré que les deux souches étudiées présentent une grande résistance face à des « stress » digestifs modérés. Toutefois, dans des conditions de « stress » élevées, la majeure partie des bactéries ne résiste pas, la souche fortement autolytique (LS 2501) étant la plus sensible.

Ces résultats démontrent la nécessité, pour obtenir un effet notable sur l'équilibre biologique de la flore du tractus intestinal de l'homme, d'ingérer une quantité importante de bactéries pour assurer une survie suffisante des souches à l'entrée du côlon.

### Influence de cultures de bactéries propioniques sur la croissance des bifidobactéries

Pour effectuer ces investigations, deux souches de bactéries bifides : *B.longum* et *B.bifidum* ont été ensemencées dans un milieu contenant, pour moitié :
- un bouillon de culture adapté à la croissance des bactéries bifides,
- une culture de bactéries propioniques (souches LS 410 ou LS 2501), soit jeunes (48 h), donc peu lysées, soit âgées de 11 jours, donc fortement lysées, notamment en ce qui concerne la souche autolytique LS 2501.

On a ainsi observé que la présence d'une culture jeune de LS 410 et, de façon moindre, de LS 2501 entraîne un niveau plus élevé de la population totale de *B.bifidum* en fin de phase exponentielle : le nombre de bactéries viables est 3 à 4 fois supérieur en présence de LS 410 et 2 à 3 fois supérieur en présence de LS 2501.

En ce qui concerne *B.longum,* la culture de LS 410 entraîne, en fin de croissance, une population 2 fois plus élevée de *B.longum.*

Dans le cas des cultures âgées, on a pu observer un effet nettement favorable des deux souches sur la croissance de *B.bifidum* lors de la phase exponentielle (population 2 à 3 fois supérieure).

En conclusion, les deux bactéries propioniques, testées dans cette étude, ont une influence sur la croissance de *B.bifidum.* Leur effet est différent selon l'âge de la culture : les cultures jeunes entraînent une augmentation de la viabilité du bifide en fin de croissance conduisant à des populations finales 2 à 4 fois plus importantes (effet plus marqué de LS 410) alors que les cultures âgées ont une action uniquement au début de la croissance en diminuant le temps de génération, mais sans modifier les niveaux de population finale.

### Caractérisation de l'agent responsable de l'effet bifidogène

- *Influence de surnageants de culture de bactéries propioniques*
   - *Rôle de l'acide propionique.*
      On a effectué des expériences similaires à celles mentionnées ci-dessus en utilisant, d'une part, uniquement les surnageants de culture constituant la fraction extracellulaire des bactéries et, d'autre part, un milieu comparatif supplémenté en acide propionique.
      On a ainsi pu observer que, comparativement à un témoin, les surnageants de culture de bactéries propioniques ont un effet nettement stimulant sur la croissance de *B.bifidum* (3 à 4 fois plus de bactéries viables après 9 h de culture) ; cet effet est plus marqué en fin de croissance avec la souche LS 2501.
      On a vérifié que ces effets promoteurs sont dus en partie à l'influence du propionate. Il apparaît toutefois que le propionate n'est pas seul responsable de ces effets, notamment dans le cas de la souche LS 2501.
      L'acide propionique possède également une influence stimulante sur l'activité métabolique de *B.longum.*
- *Influence du milieu intracellulaire :*
   On a pu observer que la présence du milieu intracellulaire des deux souches de bactéries propioniques entraîne une augmentation conséquente de la densité optique et du poids sec cellulaire des deux bifides. Toutefois, ces effets ne sont pas corrélés à une augmentation de la croissance de *B.bifidum* ou *B.longum* vu que leur viabilité ainsi que leur activité métabolique lors de la croissance ne sont pas modifiées.
- *Influence des parois isolées* :
   On a pu observer que les parois isolées de bactéries propioniques, hydrolysées ou non, n'ont pas d'influence sur la croissance de *B.longum* et *B.bifidum.*
   En conclusion, l'effet promoteur des bactéries propioniques sur la croissance des bifides n'apparaît lié ni à leur contenu intracellulaire, ni aux parois. En revanche les composés responsables sont principalement localisés dans le milieu intracellulaire. Le propionate correspond à l'un de ces composés.

## Revendications

1. Utilisation d'une préparation déshydratée renfermant des bactéries propioniques en quantité d'au moins 10⁹ cellules/g de préférence d'environ 10¹⁰ à 10¹² cellules/g pour l'obtention d'une composition diététique absorbable susceptible d'améliorer l'équilibre biologique de la flore du tractus intestinal de l'homme et des mammifères en stimulant le développement des bactéries bifides dans l'intestin.

2. Utilisation selon la revendication 1,
**caractérisée en ce que**
la composition renferme des bactéries bifides.

3. Utilisation selon la revendication 2,
**caractérisée en ce que**
la composition renferme 80 à 99 % de bactéries propioniques et 20 à 1 % de bactéries bifides.

4. Utilisation selon l'une quelconque des revendications 1 à 3,
**caractérisée en ce que**
les bactéries propioniques appartiennent à l'espèce Propioni*bacterium freudenreichii ou Propionibacterium shermanii* ou *Propionibacterium thoenii* ou *Propionibacterium jensenii* ou *Propionibacterium acidipropionici.*

5. Utilisation selon l'une quelconque des revendications 2 à 4,
**caractérisée en ce que**
les bactéries bifides appartiennent à l'espèce *Bifidobacterium* et sont choisies parmi les souches *B.bifidum, B.longum, B.adolescentis, B.breve, B.infantis* et *B.pseudolongum.*

6. Utilisation selon l'une quelconque des revendications 1 à 5,
**caractérisée en ce que**
la composition se présente sous forme de fractions individuelles d'environ 100 mg à 1 g de préférence de 200 à 500 mg renfermant la dose de bactéries devant être absorbée quotidiennement.

7. Utilisation selon la revendication 6,
**caractérisée en ce que**
la composition se présente sous forme de gélules.

8. Utilisation selon l'une quelconque des revendications 6 et 7,
**caractérisée en ce que**
chaque fraction individuelle renferme au moins 10⁹, de préférence environ 10¹⁰ à 10¹² bactéries.

## Patentansprüche

1. Verwendung einer dehydrierten Zubereitung mit Propionsäure-Bakterien in einer Menge von zumindest 10⁹ Zellen/g, vorzugsweise von etwa 10¹⁰ bis 10¹² Zellen/g, zum Erhalt einer absorbierbaren diätetischen Zusammensetzung zur Verbesserung des biologischen Gleichgewichts der Flora des Darmtraktes von Mensch und Säugern durch Stimulierung der Entwicklung von Bifidusbakterien im Darm.

2. Verwendung nach Anspruch 1, **dadurch gekennzeichnet, daß** die Zusammensetzung Bifidusbakterien enthält.

3. Verwendung nach Anspruch 2, **dadurch gekennzeichnet, daß** die Zusammensetzung 80 bis 99 % Propionsäure-Bakterien und 20 bis 1 % Bifidusbakterien enthält.

4. Verwendung nach einem der Ansprüche 1 bis 3, **dadurch gekennzeichnet, daß** die Propionsäure-Bakterien zur Spezies *Propionibacterium freudenreichii* oder *Propionibacterium shermanii* oder *Propionibacterium thoenii* oder *Propionibacterium jensenii* oder *Propionibacterium acidipropionici* gehören.

5. Verwendung nach einem der Ansprüche 2 bis 4, **dadurch gekennzeichnet, daß** die Bifidusbakterien zur Spezies *Bifidobacterium* gehören und ausgewählt sind unter den Stämmen *B.bifidum, B.longum, B.adolescentis, B.breve, B.infantis und B.pseudolongum.*

6. Verwendung nach einem der Ansprüche 1 bis 5, **dadurch gekennzeichnet, daß** die Zusammensetzung in Form einzelner Teilmengen von etwa 100 mg bis 1 g, vorzugsweise von 200 bis 500 mg, mit der täglich zu absorbierenden Bakteriendosis ausgestaltet ist.

7. Verwendung nach Anspruch 6, **dadurch gekennzeichnet, daß** die Zusammensetzung die Form von Gelkörpern aufweist.

8. Verwendung nach einem der Ansprüche 6 und 7, **dadurch gekennzeichnet, daß** jede einzelne Teilmenge zumindest 10⁹, vorzugsweise etwa 10¹⁰ bis 10¹² Bakterien enthält.

## Claims

1. Use of a dehydrated preparation containing propionic bacteria in an amount of at least 10⁹ cells/g, preferably approximately from 10¹⁰ to 10¹² cells/g, for obtaining an absorbable dietary composition capable of improving the biological balance of the flora of the intestinal tract of humans and mammals by stimulating the development of bifidus bacteria in the intestine.

2. Use according to claim 1, **characterised in that** the composition contains bifidus bacteria.

3. Use according to claim 2, **characterised in that** the composition contains from 80 to 99% of propionic bacteria and from 20 to 1% of bifidus bacteria.

4. Use according to any one of claims 1 to 3,
**characterised in that** the propionic bacteria belong to the species *Propionibacterium freudenreichii* or *Propionibacterium shermanii* or *Propionibacterium thoenii* or *Propionibacterium jensenii* or Propionibacterium *acidipropionici.*

5. Use according to any one of claims 2 to 4,
**characterised in that** the bifidus bacteria belong to the *Bifidobacterium* species and are selected from the strains *B. bifidum, B. longum, B. adolescentis, B. breve, B. infantis* and *B. pseudolongum.*

6. Use according to any one of claims 1 to 5,
**characterised in that** the composition is in the form of individual fractions of approximately from 100 mg to 1 g, preferably from 200 to 500 mg, containing the dose of bacteria that has to be absorbed daily.

7. Use according to claim 6, **characterised in that** the composition is in the form of capsules.

8. Use according to either claim 6 or claim 7,
**characterised in that** each individual fraction contains at least 10⁹, preferably approximately from 10¹⁰ to 10¹² bacteria.
